# EUROPEAN PATENT SPECIFICATION

(11) **EP 0 577 593 B1**
(45) Date of publication and mention of the grant of the patent: **26.06.1996**
(21) Application number: 91906873.4
(22) Date of filing: 28.03.1991
(51) Int. Cl.: A61J 3/07

(54) **A FILLING DEVICE FOR MEDICINE CAPSULES WITH A ROTATABLE SPIRAL SPRING MEANS**
FÜLLVORRICHTUNG FÜR MEDIZINISCHE KAPSELN MIT EINER ROTIERBAREN SPIRALFEDEREINRICHTUNG
DISPOSITIF EQUIPE D'UN RESSORT ROTATIF EN SPIRALE POUR LE REMPLISSAGE DE GELULES DESTINEES A RECEVOIR UN MEDICAMENT

(43) Date of publication of application: 12.01.1994
(73) Proprietor: LEIRAS OY, SF-20210 Turku (FI)
(72) Inventor: HARTZELL, Rolf, SF-20300 Turku (FI); HELLE, Timo, SF-20310 Turku (FI); LANKINEN, Pekka, SF-20600 Turku (FI); NIEMINEN, Pekka, SF-21540 Preitilä (FI)
(74) Representative: Haimelin, Jukka Ilmari
(86) International application number: FI9100091
(87) International publication number: WO9217149

(56) References cited:
- FR-A- 2 306 679
- GB-A- 2 237 258
- US-A- 4 731 979

## Description

The present invention relates to an equipment for filling a medical substance essentially in powder form into capsules. The capsules are intended to be implanted under the skin, and from which the medical substance diffuses to the blood circulation through the capsule wall. Intended uses are for instance dosing of contraceptive hormones, as well as in dosing antabus agents.

Equipments for said purpose are described for instance in US patent 4 731 979 and French patent application publication No 2 306 679 on which the preamble of Claim 1 is based. In the equipment disclosed in the first mentioned publication, the medical substance for each capsule is metered using an aperture plate glidable on a smooth surface. The equipment includes a mandrel for compressing the powder medicine substance into the metering apertures, and a second mandrel for feeding the medical substance from the metering apertures into capsules.

The equipment in the French publication 2 306 679 discloses a tubular madrel for metering of the medical substance for each capsule. The medical substance dose is cut by means of this madrel from a medical substance mat of a certain thickness into a "rod" of certain length and diameter. The mandrel includes a piston device glidably moovable in the hollow mandrel in order to push the metered medical substance dose from the mandrel into a capsule. Both of the aforementioned equipments have at least some compressing effect to the powder medical substance.

The equipment according to the invention is defined in Claim 1.

In the capsule production implementing the filling device of the invention of the application, the starting point is a tube for forming the casing of the capsule, and for which a suitable material is a silicone plastic. The diameter of the tube under the manufacturing of hormone capsules is 1.5 mm. The tube is fed to the capsule manufacturing line as continuous lengths from a suitable apparatus. At the starting point of the capsule manufacturing line, the tube is cut into capsule blanks of a suitable length, e.g. to lengths of about 34 mm.

The said capsule blanks are then arranged into rows formed by several blanks. One row may comprise e.g. 12 blanks. One of such rows is advantageously joined together and it forms a handling unit for the next stages of the process. In order to join this handling unit row it is possible to use a means to keep the blanks in their prescribed positions and with the help of which the handling unit row is transferred from one stage to another in the process, as well as positioned for each process stage. The said means can advantageously be a cassette of a clamping claw structure where the claws are in a mutual spring loaded clamping contact and where mutually co-operating indentation slots have been formed on the contact faces of the claws in order to form through holes for receiving the capsule blanks.

In the following process stages, the dosed quantity of the material is dropped from each dosing means to its respective transportation groove made in the surface of a disc, operating as a vibrating conveyor. The dosed material quantities proceed in the grooves of the vibrating conveyor further to feeding funnels, below which the capsule blanks to be filled have been transferred, carried by the clamping claw cassette. In order to promote the flow of the material into the capsule blanks, a special spiral feeder is used. The said feeder comprises a spiral spring that has a diameter less than the inner diameter of the capsule blank, and a straight guiding wire therein. This spring is rotatable in the forward direction of its spiral.

As far as the feeding process itself is concerned, the procedure is carried out so that the spiral spring is inserted into the capsule blank and made to rotate. Simultaneously with the activation of the rotative movement raising of the spiral spring from the capsule blank is initiated. When the rotation direction is as said above the spring acts as a feeder screw. When the filling of the capsule blank advances, the spiral spring is extracted, still in rotation, from the capsule blank. During the said operational stages, the feeding funnels are kept under vibration. When the head of the spiral spring has risen to the desired material filling level, its rising movement is stopped but vibration and rotation of the spring are continued. Thus, the spiral spring determines the height of the material column fed to the capsule.

After the filling stage, it is advisable to clean the inner surfaces of the open ends of the capsule blanks to remove any adhered filling material to ensure the sealing of the ends of the capsule blanks without problems. The cleaning may be carried out on the inside of the mouth area of the capsule blank by using a scraping rotating mandrel or a corresponding brush. The ends of the capsule blanks are sealed by using similar procedures as in connection with the sealing of the first ends of the capsule blanks described above. After the glue used in the sealing process has hardened, the capsule ends may be similarly finished by cutting parts away from their sealed portions.

After these stages the completed capsules are conveyed through as such known process stages, whereby the capsules are washed, dried, inspected, packaged and sterilized to ready-to-use products.

In the following, the invention will be described referring to the annexed drawing where:

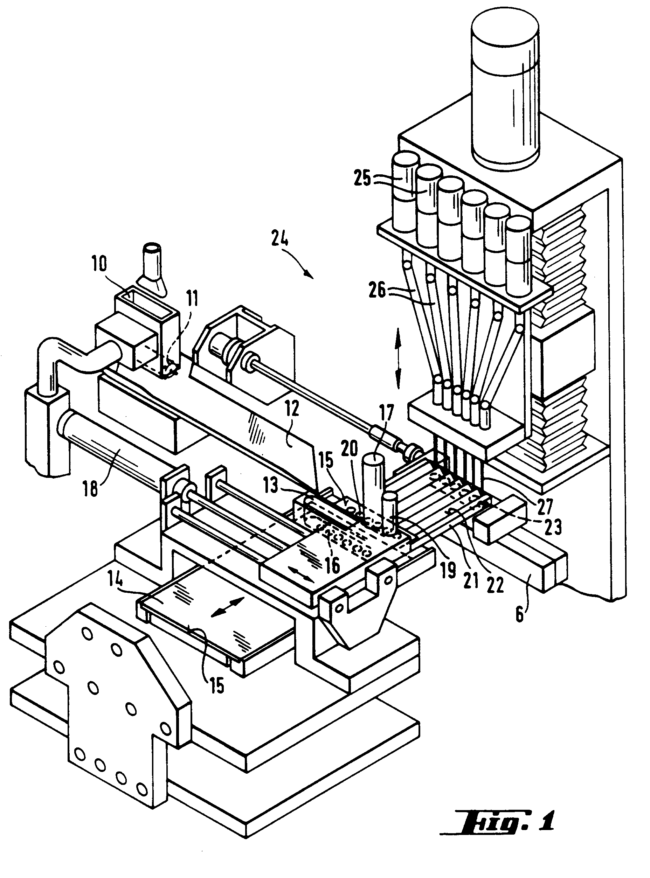

Fig. 1 shows a dosing and filling apparatus axonometrically;

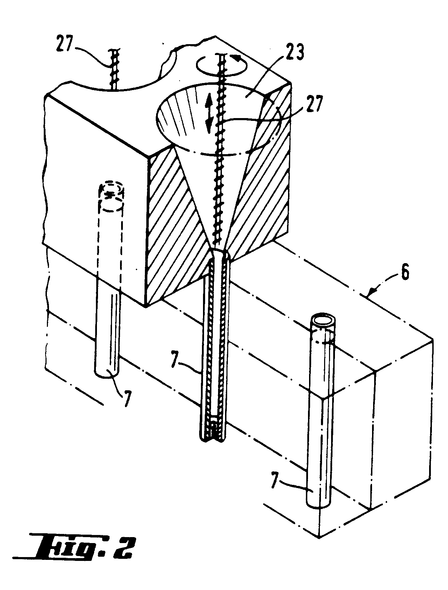

Fig. 2 shows a magnified detail from the area of the feeding funnels.

The material batches dosed on the groove disc 21 are transferred to filling funnels 23 placed in a transverse row below the tail edge of the groove disc 21. Beneath these filling funnels, a row of capsule blanks 7 carried by a clamping claw cassettes 6 has been conveyed the lower ends of which capsules have been sealed and advantageously trimmed.

A filling device 24 comprises feeding devices, the number of which corresponds to the number of the filling funnels 23. Each feeding device comprises a rotating motor 25, a spindle 26, and a feeder screw 27 attached to the the lower end of the spindle. To operate the feeder screw 27 within the conditions determined by the small dimensions of the capsules to be filled, the filling screw is formed by a thin spiral spring. For better control of the movements of the spiral spring, another thin straight guiding wire is placed inside the spring, extending substantially along the whole length of the spiral spring.

The feeder screw 27 formed by the spiral spring is directed through each feeding funnel 23 to the capsule blank below it and is made to rotate. The rotative movement is naturally chosen in the feeding direction of the spiral spring. The feeder screw rotating in the capsule blank feeds the material fed into the feeder funnel efficiently into the capsule blank. In order to avoid unnecessary compacting of the material in the capsule blank, the feeder screw is raised as filling is advancing. After the dosed batch has been completely fed, the feeder screw is removed completely from the capsule blank and the blanks are transferred to be sealed. Before sealing it is possible to clean the inside of their mouth areas to remove any remaining filling material. The sealing is carried out by glueing in the same way as sealing of the opposite end of the blanks before the filling stage.

After sealing, the ends of the filled capsules can be trimmed by cutting. After trimming, the completed capsules will be removed from their respective clamping claw cassettes after which operation they will be processed individually according to prior technology discussed briefly above.

## Claims

1. Equipment for filling medicine capsules, the said capsules being arranged as blanks (7), with closed lower ends and open upper ends, vertically into rows comprising a certain number of capsules, which equipment (24) comprises a device dosing a desired quantity of the medicine for each capsule and a device (25, 26) forwarding the dose to the actual capsule blank, **characterized** in that for feeding of the medicine dose into the actual capsule blank (7) the equipment further comprises a funnel (23) placed above each capsule blank, and a rotatable spiral spring means (27) insertable through the funnel into the capsule blank.

## Patentansprüche

1. Vorrichtung zum Füllen von medizinischen Kapseln, die als an ihren unteren Enden geschlossenen und an ihren oberen Enden offenen, senkrechtstehende Vorformlinge (7) in eine gewisse Anzahl von Kapseln umfassenden Reihen angeordnet sind, welche Vorrichtung (24) eine Einrichtung zum Dosieren einer gewüschten Menge des Arzneimittels in jede Kapsel und eine Einrichtung (25, 26) zum Befördern der Dosis zu dem jeweiligen Kapselvorformling aufweist, dadurch **gekennzeichnet,** daß die Vorrichtung zum Zuführen der Arzneimitteldosis zu dem jeweiligen Kapselvorformling (7) zusätzlich einen über jedem Kapselvorformling angeordneten Trichter (23) und eine rotierbare, durch den Trichter in den Kapselvorformling einzusetzende Spiralfedereinrichtung (27) aufweist.

## Revendications

1. Installation de remplissage de gélules de médicament, lesdites gélules étant disposées, sous la forme de capsules (7) avec des extrémités inférieures fermées et des extrémités supérieures ouvertes, verticalement en rangées comprenant un certain nombre de gélules, laquelle installation (24) comprend un dispositif dosant une quantité souhaitée du médicament pour chaque gélule, et un dispositif (25, 26) pour amener la dose dans la capsule (7) présente, caractérisée en ce que, pour amener la dose de médicament dans la capsule présente, l'installation comprend de plus un entonnoir (23) placé au-dessus de chaque capsule, et un moyen à ressort en spirale rotatif (27) pouvant être inséré par l'entonnoir dans la capsule.
